(19) Europäisches Patentamt
European Patent Office
Office européen des brevets

(11) Publication number: 0 337 616
A1

(12) # EUROPEAN PATENT APPLICATION

(21) Application number: 89302772.2

(22) Date of filing: 21.03.89

(51) Int. Cl.4: C07D 277/82 , C07D 277/70 , C07D 277/68 , A01N 43/78

(30) Priority: 15.04.88 GB 8808970

(43) Date of publication of application:
18.10.89 Bulletin 89/42

(84) Designated Contracting States:
BE DE ES FR GB GR IT NL

(71) Applicant: SCHERING AGROCHEMICALS LIMITED

Hauxton Cambridge CB2 5HU(GB)

(72) Inventor: Mellor, Michael
2 Biscay Close
Haverhill Suffolk(GB)
Inventor: Steele, Christopher Richard
4 Twyford Road
Bishops Stortford, Herts(GB)
Inventor: Holah, David Stanley
4 Green End Farm Cottages, Six Mile Bottom Road
West Wratting Cambridge(GB)

(74) Representative: Waldman, Ralph David et al
Schering Agrochemicals Limited Industrial Property Department Chesterford Park Research Station
Saffron Walden Essex CB10 1XL(GB)

(54) ## Benzthiazole fungicides.

(57) Compounds of formula I

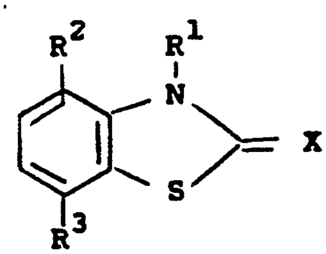

(I)

where $R^1$ is alkyl, alkenyl or alkynyl, each of which is optionally substituted;
$R^2$ and $R^3$, which may be the same or different, have rhe same meaning as $R^1$ or are halogen; and
X is O, S or $NR^4$, where $R^4$ is hydrogen or acyl, have valuable fungicidal activity, especially against rice blast.

EP 0 337 616 A1

## Benzthiazole Fungicides

This invention relates to compounds having fungicidal activity.

According to the invention there is provided a compound of formula I

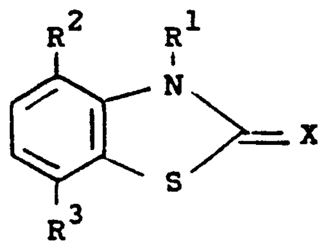

(I)

where $R^1$ is alkyl, alkenyl or alkynyl, each of which is optionally substituted;
$R^2$ and $R^3$, which may be the same or different, have the same meaning as $R^1$ or are halogen; and
X is O, S or $NR^4$, where $R^4$ is hydrogen or acyl.

Alkyl groups are preferably of 1 to 4 carbon atoms, especially methyl, and alkenyl and alkynyl groups are usually of 3 to 4 carbon atoms. Optional substituents, when present on any alkyl, alkenyl or alkynyl group, are preferably halogen or alkoxy (e.g. of 1 to 4 carbon atoms). Other substituents include alkylthio, cyano, nitro, optionally substituted amino, carboxy, alkoxycarbonyl, acyl, acyloxy and aryl. Aryl groups are usually phenyl, optionally substituted, e.g. by halogen, alkyl, haloalkyl, alkoxy or nitro. The term aryl may include heteroaryl groups such as thienyl, furyl or pyridyl. The term 'acyl' includes the residue of sulphonic and phosphorus containing acids as well as carboxylic acids. Acyl groups are preferably alkanoyl e.g. of 1 to 4 carbon atoms. Amino groups may be substituted, e.g. by one or two alkyl or acyl groups or two substituents that can form a ring. e.g. a morpholino or piperidino ring.

Particularly preferred compounds of the invention are those where:
$R^1$ is alkyl, epecially methyl;
$R^2$ and $R^3$, which may be the same or different, are alkyl, especially methyl, or halogen, especially chlorine; and
X is O or NH, especially NH.

The compounds of the invention have activity as fungicides, especially against fungal diseases of plants, and especially diseases of rice such as rice blast (Pyricularia oryzae). Other diseases against which the compounds may be active include mildews and particularly barley powdery mildew (Erysiphe graminis) and vine downy mildew (Plasmopora viticola) and blight of potatoes or tomatoes (Phytophthora infestans).

Many of the compounds, especially those where X is NH, have systemic activity, especially against Pyricularia oryzae in rice; ie compound is taken up through the roots and helps prevent infection developing in other parts of the plant.

The invention thus also provides a method of combating fungi at a locus infested or liable to be infested therewith, which comprises applying to the locus a compound of formula I.

The invention also provides an agricultural composition comprising a compound of formula I in admixture with an agronomically acceptable diluent or carrier.

The composition of the invention may of course include more than one compound of the invention.

In addition the composition can comprise one or more additional active ingredients, for example compounds known to possess plant-growth regulant, herbicidal, fungicidal, insecticidal or acaricidal properties. Alternatively the compounds of the invention can be used in sequence with the other active ingredient.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more

complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7, 9-tetramethyl-5-decyn-4,7-diol, or ethoxylated acetylenic glycols.

Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of agrochemicals, for example, a solution, a dispersion, an aqueous emulsion, a dusting powder, a seed dressing, a fumigant, a smoke, a dispersible powder, an emulsifiable concentrate or granules. Moreover it can be in a suitable form for direct application or as a concentrate or primary composition which requires dilution with a suitable quantity of water or other diluent before application.

An emulsifiable concentrate comprises a compound of the invention dissolved in a water-immiscible solvent which is formed into an emulsion with water in the presence of an emulsifying agent.

A dusting powder comprises a compound of the invention intimately mixed and ground with a solid pulverulent diluent, for example, kaolin.

A granular solid comprises a compound of the invention associated with similar diluents to those which may be employed in dusting powders, but the mixture is granulated by known methods. Alternatively it comprises the active ingredient absorbed or adsorbed on a pre-granular diluent, for example, Fuller's earth, attapulgite or limestone grit.

Wettable powders, granules or grains usually comprise the active ingredient in admixture with a suitable surfactant and an inert powder diluent such as china clay.

Another suitable concentrate is a flowable suspension concentrate which is formed by grinding the compound with water or other liquid, a wetting agent and a suspending agent.

The concentration of the active ingredient in the composition of the present invention, as applied to plants is preferably within the range of 0.01 to 3.0 per cent by weight, especially 0.01 to 1.0 per cent by weight. In a primary composition the amount of active ingredient can vary widely and can be, for example, from 5 to 95 per cent by weight of the composition.

In the method of the invention the compound is generally applied to seeds, plants or their habitat. Thus, the compound can be applied directly to the soil or paddy, in the case of paddy grown rice, before, at or after sowing. The active compound can be applied in any manner which allows it to be intimately mixed with the growing medium such as by spraying or by broadcasting a solid form eg of granules or dusts. A suitable applications rate is within the range of from 0.05 to 20 kg per hectare, more preferably from 0.1 to 10 kg per hectare.

Alternatively the active compound can be applied directly to the plant by, for example, spraying or dusting either at the time when the fungus has begun to appear on the plant or before the appearance of fungus as protective measure. In both such cases the preferred mode of application is by foliar spraying. It is generally important to obtain good control of fungi in the early stages of plant growth as this is the time when the plant can be most severely damaged. The spray or dust can conveniently contain a pre- or post-emergence herbicide if this is thought necessary. Sometimes, it is practicable to treat the roots of a plant before or during planting, for example, by dipping the roots in a suitable liquid or solid composition. When the active compound is applied directly to the plant a suitable rate of application is from 0.01 to 10 kg. per hectare, preferably from 0.05 to 5 kg per hectare.

The compounds of the invention may be prepared in a variety of ways, e.g. by reacting a compound of formula II

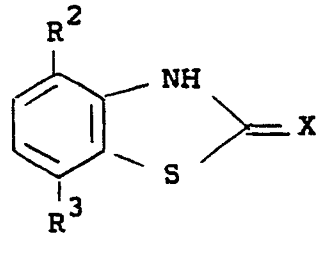

(II)

with a compound $R^1O$, where Q is a leaving group, such as halogen and especially iodine, or $-OSO_2OR^1$, under basic conditions. The compounds of formula II, in which X is oxygen, can be prepared by hydrolysis of a compound of formula III

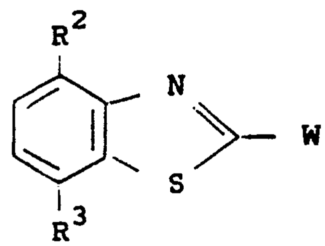

(III)

where W is a halogen group. Compounds of formula III can be obtained in known manner as for example by methods described in British Patent 1,397,089.

Compounds of formula I where X is NH can be obtained from a compound of formula III, where W is amino, by reaction with a compound of formula $R^1Q$, where Q is as defined above. The imino group can then be substituted in known manner, e.g. by treatment with a compound of formula $R^4Q$, where $R^4$ is acyl and Q is as defined above.

Compounds where X is sulphur can be obtained from compounds where X is oxygen by treatment with a sulphurising agent, e.g. Lawesson's reagent.

The invention is illustrated in the following Examples. Structures of isolated novel compounds were confirmed by elemental and/or other appropriate analyses. Temperatures are in °C.

Example 1

Benzoyl chloride (21.9 g) was added dropwise to a stirred mixture of ammonium thiocyanate (13.2 g) in acetone (95 ml) and the mixture stirred for a further 20 minutes at room temperature. A solution of 6-chloro-m-toluidine (22.1 g) in acetone (30 ml) was added dropwise with stirring, the mixture stirred for a further 30 minutes at room temperature and then heated under reflux for 5 minutes. The mixture was added to water, the solid collected, dissolved in aqueous sodium hydroxide (10%; 95 ml) acidified to pH 5 with hydrochloric acid and rebasified to pH 9 with aqueous ammonia. The solid was filtered and recrystallised from toluene to give 1-(6-chloro-m-tolyl)thiourea, m.p. 152-4°. Bromine (22.17 g) was added, dropwise to a stirred suspension of the crude thiourea (18.54 g) in acetic acid (18.7 ml) and 1,2-dichloroethane (105 ml) and the resulting solution refluxed for 3 hours. After cooling, the reaction mixture was diluted with diethyl ether and the precipitated solid collected by filtration, washed with diethyl ether and suspended in water, treated with sodium bicarbonate until no more carbon dioxide evolved, filtered and dried to give the crude intermediate, 2-amino-4-chloro-7-methyl-benzothiazole. This product (21 g) was dissolved in dichloromethane (100 ml) and hydrochloric acid (55 ml) and sodium nitrite (21.9 g) added, portionwise, to the stirred mixture whilst maintaining a reaction mixture temperature of <5°. After stirring for a further one hour at 0-5° and overnight at room temperature, the reaction mixture was filtered. The organic phase of the filtrate was separated, washed with water, dried, filtered through a bed of silica gel and evaporated to give crude 2,4-dichloro-7-methylbenzothiazole, which was used without further purification. A mixture of this crude product (11.1 g), concentrated sulphuric acid (25 ml) and methanol (190 ml) was stirred at reflux for 5 hours. After cooling, the reaction mixture was poured into water and the solid produced, collected by filtration, washed well with water and dried and recrystallised from toluene to give crude 4-chloro-7-methyl-2(3H)-benzothiazolone. The product was used without further purification. A mixture of this crude product (2.01 g) and anhydrous potassium carbonate (1.39 g) in 2-butanone (50 ml) was stirred at reflux for 1 hour. Iodomethane (5.34 g) was then added and the reaction mixture stirred at reflux for a further 2 hours. After cooling, the reaction mixture was filtered and the filtrate evaporated. The residue was recrystallised from cyclohexane to give 4-chloro-3,7-dimethyl-2(3H)-benzothiazolone, m.p. 126-8°. (Compound 1)

Example 2

A mixture of 2-amino-4,7-dichlorobenzothiazole (3.21 g) and dimethyl sulphate (2.03 g) was heated at 150° for 2 hours. The resulting mixture was then worked up in conventional manner to give 4,7-dichloro-3-methyl-benzothiazolimine, m.p. 127-129°. (Compound 2)

Example 3

In a similar manner to the method described in the previous Examples the following compounds were obtained

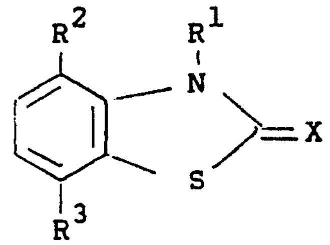

| Cpd No | X | $R^1$ | $R^2$ | $R^3$ | m.p. |
|---|---|---|---|---|---|
| 3 | O | Me | Me | Me | 103-4 |
| 4 | O | Me | Cl | Cl | 136-8 |
| 5 | O | Me | Me | Cl | 114-6 |
| 6 | O | Me | Br | Br | 158-160 |
| 7 | O | Et | Cl | Me | 80-2 |
| 8 | NH | Me | Cl | Me | 93 |
| 9 | NH | Me | Cl | Br | 130-132 |

Example 4

This example illustrates typical concentrates that can be formulated from compounds of the invention.

| Wettable powder | |
|---|---|
| Compound of the invention | 25% w/w |
| Reax 45L* | 10% w/w |
| China clay | 65% w/w |

* = mixture of sodium lignosulphonate and an anionic wetting agent.

Granule

| | |
|---|---|
| Compound of the invention | 2% w/w |
| Gypsum granules | 98% w/w |

Test Example

The compounds of the invention were assessed for activity against Pyricularia oryzae: rice blast

a) Foliar application

Aqueous solutions or dispersions of the compounds at the desired concentration, including a wetting

agent, were sprayed onto rice plants and then inoculated by spraying with spore suspensions of the fungi. Plants were then kept under controlled environment conditions suitable for maintaining plant growth and development of the disease. After an appropriate time, the degree of infection of the leaf surface was visually estimated.

Compounds were considered active if they gave greater than 50% control of the disease at a concentration of 25 ppm (w/v) or less. Compounds 1 to 9 were all active in this test.

b) Systemic test

Aqueous solutions or dispersions of the compounds at the desired concentration, including a wetting agent, were applied as a drench (20 ml) to the soil of pots (volume 150 ml) each containing rice plants at the 1-2 leaf stage. Immediately after treatment the pots are placed in the extraction hood of a spray room so that there is a constant stream of air being drawn over the soil surface and extracted in such a way that no vapour is allowed to come into contact with the leaves. This is to ensure that any compound reaching the leaf only does so by systemic action. 48 hours after treatment, sections of leaf are removed from the plants and placed on 0.5% agar in plastic boxes. Spore suspensions of the fungi were then sprayed onto the leaves and the boxes kept under controlled environment conditions suitable for development of the disease. After an appropriate time, the degree of infection of the leaf surface was visually estimated.

Results are shown below:

| Compound No | Rate (ppm in pot) | % control |
|---|---|---|
| 2 | 20 | 88 |
| | 10 | 64 |
| 8 | 20 | 92 |
| | 10 | 48 |

## Claims

1) A compound of formula I

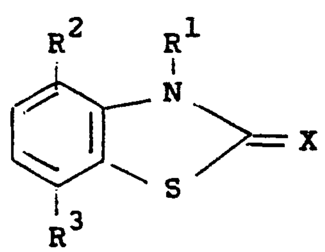

(I)

where $R^1$ is alkyl, alkenyl or alkynyl, each of which is optionally substituted:
$R^2$ and $R^3$, which may be the same or different, have the same meaning as $R^1$ or are halogen; and
X is O, S or $NR^4$, where $R^4$ is hydrogen or acyl.

2) A compound according to claim 1, where
$R^1$ is alkyl;
$R^2$ and $R^3$, which may be the same or different, are alkyl or halogen; and
X is O or NH.

3) A compound according to claim 2, where
$R^1$ is methyl;
$R^2$ and $R^3$, which may be the same or different, are methyl or chlorine; and
X is NH.

4) A fungicidal composition comprising a compound as claimed in any one of claims 1 to 3, in admixture with an agriculturally acceptable diluent or carrier.

5) A method of combating a fungus at a locus infected or liable ro be infected therewith, which comprises applying to the locus a fungicidally effective amount of a compound as claimed in any one of claims 1 to 3.

European Patent Office

# EUROPEAN SEARCH REPORT

Application Number

EP 89 30 2772

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X<br><br><br>Y | EP-A-0 040 398 (HOECHST AG)<br>* page 4, line 31 - page 6, line 38; page 14, example 33 *<br>--- | 1-3<br><br><br>4,5 | C 07 D 277/82<br>C 07 D 277/70<br>C 07 D 277/68<br>A 01 N 43/78 |
| Y | DERWENT PUBLICATIONS LTD. 10 January 1950, London, GB; 80-002780 (01) & JP - A - 552621 (SUMITOMO CHEMICAL K. K.); & PATENT ABSTRACTS OF JAPAN vol. 4, no. 26 (C-1) (508), 6 March 1980<br>--- | 4,5 | |
| X | JOURNAL OF MEDICINAL CHEMISTRY vol. 18, no. 3, 1975, pages 315-318; S. W. HORGAN et al.: "Hypotensive Activity of 3-Alkyl-2-iminobenzothiazolines" * table I, component no. 24 *<br>--- | 1-3 | |
| A | EP-A-0 245 991 (SCHERING AGROCHEMICALS LTD.)<br>* claims *<br>--- | 1-5 | TECHNICAL FIELDS SEARCHED (Int. Cl.4) |
| A | DE-A-2 801 868 (SUMITOMO CHEMICAL CO. LTD.)<br>* claims 1-3,5-8,11,12,25-29 *<br>--- | 1,4,5 | C 07 D 277/00<br>A 01 N 43/00 |
| A | FR-A-2 357 553 (BASF AG)<br>* page 1, lines 1-26 *<br>--- | 1 | |
| A | DE-A-2 206 575 (AGRIPAT S. A.)<br>* page 25, lines 4,5 *<br>--- | 1 | |
| A | US-A-3 334 990 (W. SCHAEFER et al.)<br>----- | | |

The present search report has been drawn up for all claims

| Place of search | Date of completion of the search | Examiner |
|---|---|---|
| BERLIN | 10-07-1989 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)